# EUROPÄISCHE PATENTSCHRIFT

(11) **EP 2 793 917 B1**
(45) Veröffentlichungstag und Bekanntmachung des Hinweises auf die Patenterteilung: **15.04.2020**
(21) Anmeldenummer: 12818499.1
(22) Anmeldetag: 19.12.2012
(51) Int. Cl.: A61K 36/22, A61P 1/06, A61P 17/00, A61P 1/00, A23L 33/105

(54) **EXTRAKT AUS RHUS COPALLINA ZUR VERWENDUNG ALS ARZNEIMITTEL**
EXTRACT OF RHUS COPALLINA EXTRACT FOR USE AS A MEDICAMENT
EXTRAIT DE RHUS COPALLINA POUR UTILISATION COMME MÉDICAMENT

(30) Priorität: 19.12.2011 EP 11194249
(43) Veröffentlichungstag der Anmeldung: 29.10.2014
(73) Patentinhaber: Abhau, Annette, 28857 Syke (DE); Schlenk, Margit, 91230 Happurg-Foerrenbach (DE)
(72) Erfinder: ABHAU, Annette, 28857 Syke (DE)
(74) Vertreter: Simandi, Claus
(86) Internationale Anmeldenummer: PCT/EP2012/076226
(87) Internationale Veröffentlichungsnummer: WO 2013/092758

(56) Entgegenhaltungen:
- Annette Abhau: "The survival of a Malagasy lemur species Propithecus verreauxi coquereli in captivity: The vital role of a self-selected plant diet", , 20. Juni 2007 (2007-06-20), Seiten 1-325, XP055030205, Gefunden im Internet: URL:http://d-nb.info/984680519/34 [gefunden am 2012-06-18] in der Anmeldung erwähnt
- MA HANG ET AL: "Identification of bioactive compounds from New England plants", ABSTRACTS OF PAPERS AMERICAN CHEMICAL SOCIETY, Bd. 238, August 2009 (2009-08), Seite 162, XP009160324, & 238TH AMERICAN-CHEMICAL-SOCIETY NATIONAL MEETING; WASHINGTON, DC, USA; AUGUST 16 -20, 2009 ISSN: 0065-7727
- N.N.: "Winged Sumac (Rhus copallina)", , 4. September 2010 (2010-09-04), XP055030183, Gefunden im Internet: URL:http://edibleplantproject.org/sumac/ [gefunden am 2012-06-18]
- E Teuscher: "Gerbstoffe" In: "Biogene Arzneimittel", 1 January 1997 (1997-01-01), Wissenschaftliche Verlagsgesellschaft mbH, XP055622008, pages 224-225,
- R Hänsel ET AL: "Gerbstoffe" In: "Pharmakognosie - Phytopharmazie", 1 January 2003 (2003-01-01), Springer, XP055622040, pages 1249-1250,

## Beschreibung

Die Erfindung betrifft Arzneimittel oder Nahrungsergänzungsmittel umfassend einen wässrig-ethanolischen Extrakt aus Blättern von Rhus copallina L. zur Anwendung in Verfahren gemäß den Ansprüchen.

Es besteht ein großer Bedarf an neuen Arzneimitteln oder Nahrunsgergänzungsmitteln zur Behandlung von gastrointestinalen Erkrankungen.

Die Pflanzengattung Rhus gehört mit 150 bis 250 Arten zur Familie der Anacardiaceae. Viele Arten der Gattung Rhus sind von wirtschaftlicher Bedeutung bei der Herstellung von Gerb- und Färbemitteln. Alle Arten sind mehr oder weniger giftig. Viele Rhus Arten können Hautreizungen hervorrufen.

US 531,610 offenbart die Verwendung von wasserabweisenden Deckschichten und Decklacken, die das Harz und/oder den Saft von Rhus copallina enthalten.

US 7,528,232 B2 offenbart Verfahren und Stoffzusammensetzungen, die die Vermehrung von Pathogenen verhindern. Dabei werden einzelne Proteine, die aus Pflanzen extrahiert werden, als Antibiotikum gegen humane und tierische Pathogene eingesetzt. Gemäß US 7,528,232 können entsprechende endogene Proteine aus Pflanzen, beispielsweise aus Rhus copallina, durch Screeningverfahren erhalten werden. Ein konkretes Verfahren für Rhus copallina oder ein konkretes Protein aus Rhus copallina werden nicht offenbart.

US 7,501,557 B1 betrifft ein System, mit dem Proteine und Proteinprodukte an die Oberfläche von Pflanzen transportiert werden und ein Verfahren, um Proteine aus der Pflanze abzusondern. US 7,501,557 B1 schlägt vor, dazu beispielsweise Rhus copallina mit dem in US 7,50 1,557 B1 offenbarten Genkonstrukt zu versehen.

Die Dissertation von Annette Abhau zum Thema "The survival of a Malagasy lemur species Propithecus verreauxi coquereli in captivity : The vital role of a self-selected plant diet" (freigeschaltet am 20.06.2007; http://deposit.d-nb.de/cgi-bin/dokserv?idn=984680519 beschäftigt sich u.a. mit der genauen qualitativen und quantitativen Analyse der Pflanze Rhus copallina als essentielles Nahrungsmittel für die genannte Lemurenart, auch als Sifakas bezeichnet. In der Doktorarbeit wurden für die Analysen und Untersuchungen die stiellosen Blätter verwendet. Der Nährstoffgehalt der Blätter wurde nach einer Methode von Weender bestimmt. Zur Analyse der Inhaltsstoffe von Rhus copallina wurden die Blätter von Rhus copallina mit einem Gemisch aus 80 % Methanol und 20 % Wasser (V/V) extrahiert. Vor der präparativen Isolierung einzelner Verbindungen mit Hilfe einer Polyamidsäule und nachfolgender präparativer HPLC-Technik wurden die zerkleinerten Blätter mit n-Hexan präextrahiert.

Für eine gravimetrische Isolierung wurde Isobutylmethylketon und Wasser verwendet.

John K. Crellin and Jane Philpott (A Reference Guide to Medicinal Plants, 1989 Duke University Press, 2. Auflage 1997, Seiten 417 - 419) geben einen Überblick über die Verwendung von verschiedenen Rhus-Arten als Hausmittel.

Für R. glabra L. und R. copallina sind beschrieben
a) die Verwendung als Arzneitee, der durch Kochen der Beeren in Wasser hergestellt wird, als Hustensaft, zum Gurgeln und für die Nieren;
b) die Verwendung der Blätter äußerlich bei Schwellungen und Geschwüren sowie als Duft- und Geschmacksstoff für Tabak und als Farbe, und
c) die Verwendung der Beeren (im Mixer zerkleinert, angefärbt und mit Wasser und Zucker versetzt) als Limonadenersatz und bei Erkältung.

John K. Crellin and Jane Philpott beschreiben ebenfalls die medizinische Verwendung von Rhus coriaria und der verwandten Cotinus coggygria Scop. als Bindemittel, zur Trocknung und bei Erkältung. Diese Anwendungen sind bereits seit dem Jahr 1597 bekannt. Für Rhus coriaria ist auch die Verwendung für "fluxes of the belly" und Diarrhö bekannt. Ferner wird die Verwendung als Fiebermittel erwähnt, sowie die kühlenden und adstringierenden Eigenschaften der Beeren. Auch die adstringierenden Eigenschaften der Blätter, Wurzeln und Beeren von R. coriaria, deren Verwendung bei rauhem Hals und Affektionen der Mundschleimhaut, bei Diarrhö, Verbrennungen und Hämorrhoiden im Zusammenhang mit R. coriaria, sowie die Verwendung der Blätter und Beeren von R. glabra bei gastrointestinalen Beschwerden gehören zur traditionellen Anwendung dieser Rhus-Arten.

Rhus glabra. Rhus typhina und Rhus aromatica sind als traditionelle nordamerikanische Medizin bekannt, und zwar als Adstringens, bei Verdauungsbeschwerden, zum Gurgeln und Mundspülen, sowie bei Beschwerden von Haut und Blase. Als Pflanzenteile werden hier nicht die Blätter verwendet, sondern nur die Rinde, Wurzeln und Früchte (In "Medicinal Plants of the world" von Ben-Erik van Wyk und Michael Wink).

Von einigen Arten (beispielsweise Toxicodendron-Arten) werden Hautreizungen hervorgerufen.

Gegenstand der Erfindung ist ein Arzneimittel oder Nahrungsergänzungsmittel umfassend einen wässrig-ethanolischen Extrakt aus Blättern von Rhus copallina L. zur Anwendung in einem Verfahren zur Behandlung von atopischer Dermatitis (Neurodermitis) oder Spasmen.

Gegenstand der Erfindung ist ebenfalls ein Arzneimittel oder Nahrungsergänzungsmittel umfassend einen wässrig-ethanolischen Extrakt aus Blättern von Rhus copallina L. zur Anwendung in einem Verfahren zur Behandlung von gastrointestinalen Erkrankungen, wobei eine gastrointestinale Erkrankung ausgewählt ist aus der Gruppe a.) chronischen Magen- und/oder Darmfunktionsstörungen, wie die Regulation desMagen-Darm Traktes, allgemeine Verdauungsprobleme, Blähungen, Koliken und/odergegen Verstopfung, Magenprobleme, wie Völlegefühl und Unwohlsein, Magenund/oder Bauchschmerzen, b.) chronisch-entzündliche Dannerkrankungen, wie Morbus Crohn oder Colitisulcerosa, Gastroenteritis, Hämorrhoidalleiden, Reizdarm.

Gegenstand der Erfindung ist ebenfalls ein Arzneimittel oder Nahrungsergänzungsmittel zur Anwendung wie beschrieben mittels einer oralen, dermalen und/oder mucosalen Darreichung.

Gegenstand der Erfindung ist ebenfalls ein Arzneimittel oder Nahrungsergänzungsmittel zur Anwendung wie beschrieben, in Form einer Salbe, Saft, Lotion oder Zäpfchen, umfassend gängige Hilfs- und Zusatzstoffe.

In der Erfindung wird der Extrakt dadurch hergestellt, dass die Blätter von Rhus copallina L. mit Ethanol und Wasser extrahiert werden. Offenbart wird auch ein Extrakt dadurch hergestellt, dass die Blätter von Rhus copallina L. mit Methanol und Wasser extrahiert werden. Die Extraktion kann beispielsweise einem Gemisch, das mindestens 20 % oder 30 % Ethanol/Methanol umfasst, vorzugsweise 40 % oder 50 % Ethanol/Methanol, besonders bevorzugt 60 %, 70 % oder 80% Ethanol oder Methanol. Offenbart wird ein Arzneimittel oder Nahrungsergänzungsmittel, wobei die Extraktion mit

einem Gemisch aus Alkohol und Wasser durchgeführt wird, beispielsweise einem Gemisch mit mindestens 85 % Ethanol oder Methanol, vorzugsweise 90 % oder 95 % Ethanol oder Methanol oder mehr, bis hin zur Verwendung von reinem Ethanol oder Methanol für die Extraktion. In einer besonderen Ausführungsform der Erfindung kann das Extraktionsmittel neben Ethanol und Wasser noch bis zu 10 % andere Lösungsmittel enthalten, beispielsweise Ethylacetat. Erfindungsgemäß ist Ethanol bevorzugt.

Das Arzneimittel oder Nahrungsergänzungsmittel ist geeignet für Tiere, insbesondere Nutztiere, und vorzugsweise Menschen. Eine bevorzugte Ausführungsform der Erfindung betrifft das Arzneimittel oder Nahrungsergänzungsmittel zur (spezifischen) Anwendung am Menschen. Eine weitere besondere Ausführungsform betrifft das Arzneimittel zur (spezifischen) Anwendung am Menschen, die unter chronischen Magen- und/oder Darmfunktionsstörungen leiden.

Die Erfindung betrifft daher die Anwendung des Arzneimittels zur Behandlung von gastrointestinalen Erkrankungen, insbesondere chronischen Magen- und/oder Darmfunktionsstörungen umfassend die Regulation des Magen-Darm Traktes, bei allgemeinen Verdauungsproblemen, beispielsweise gegen Blähungen, gegen Koliken und/oder gegen Verstopfung, bei Magenproblemen, wie Völlegefühl und Unwohlsein, Magen- und/oder Bauchschmerzen.

Eine weitere besondere Ausführungsform der Erfindung betrifft die spezifische Anwendung bei Kindern ab dem Säuglingsalter. Eine weitere besondere Ausführungsform der Erfindung betrifft die Anwendung bei Menschen, die immundefizient sind, an einer bakteriellen Fehlbesiedlung des Darmes leiden oder deren Erkrankung mit einer erhöhten gastrointestinalen Permeabilität assoziiert ist beispielsweise entzündliche Darmerkrankungen wie M. Crohn, Colitis ulcerosa, Diabetes, Asthma, allergische Hauterkrankungen, atopische Dermatitis (Neurodermitis).

Besonders bevorzugte Ausführungsformen der Erfindung betreffen Arzneimittel zur (spezifischen) Anwendung als Mittel zur Behandlung von atopischer Dermatitis (Neurodermitis), Spasmen (Spasmolytikum).

Überraschenderweise wurde gefunden, dass ein Extrakt, insbesondere ein wäßrigalkoholischer Extrakt aus den Blättern von Rhus copallina antiphlogistische, antiinflammatorische (entzündungshemmende), juckreizstillende, adstringierende, antiseptische und antibakterielle Eigenschaften aufweist.

Ein weiterer Gegenstand der Erfindung ist insbesondere die (spezifische) Anwendung des Arzneimittels oder Nahrungsergänzungsmittels zur Behandlung von gastrointestinalen Erkrankungen.

Die Erfindung betrifft die Anwendung des Arzneimittels oder Nahrungsergänzungsmittels zur Behandlung und Prophylaxe von gastrointestinalen Erkrankungen, umfassend chronisch-entzündliche Darmerkrankungen wie Morbus Crohn, Colitis ulcerosa, Diarrhö, Gastroenteritis, Hämorrhoidalleiden, Reizdarm,.

Offenbart wird auch die Anwendung des Arzneimittels oder Nahrungsergänzungsmittel zur Reduktion einer erhöhten gastrointestinalen Permeabilität.

Die erfindungsgemäßen (spezifischen) Anwendungen umfassen die Verwendung des Arzneimittels zur Behandlung und/oder Prophylaxe und/oder Nachsorge, der jeweils genannten Indikationen.

Die Definitionen und Begriffe "Magen- und Darmfunktionsstörungen, chronisch-entzündliche Darmerkrankungen, Diarrhö, Gastroenteritis, Hämorrhoidalleiden, Reizdarm, Morbus Crohn, Cholera, Verdauungsprobleme, wie Blähungen, Koliken und/oder gegen Verstopfung, Magenproblemen, wie Völlegefühl und Unwohlsein, Magen- und/oder Bauchschmerzen" können beispielsweise dem Klinischen Wörterbuch Pschyrembel, Auflage 2012, De Gruyter Verlag, Berlin entnommen werden.

Das Arzneimittel kann oral, rektal, dermal oder mucosal dargereicht werden. Eine besonders bevorzugte Ausführungsform der Erfindung betrifft die orale, dermale oder mukosale Darreichung bzw. Applikation des Arzneimittels.

Offenbart wird auch ein Verfahren zur Herstellung eines Extrakts aus Rhus copallina, wobei die Blätter von Rhus copallina geerntet, gereinigt und zerkleinert werden und die Blätter dann mit einem Gemisch aus Wasser und Alkohol, beispielsweise Ethanol oder Wasser extrahiert werden, beispielsweise mit einem Gemisch mit mindestens 40 % Alkohol, vorzugsweise 50 % Alkohol oder mehr, besonders bevorzugt 60 % oder 80 % Alkohol der mehr. In einer besonderen Ausführungsform der Erfindung kann das Extraktionsmittel neben Ethanol und Wasser noch bis zu 10 % andere Lösungsmittel enthalten.

Die Extraktion mit Alkohol und Wasser ist besonders vorteilhaft weil dadurch aus der Vielzahl der Inhaltsstoffe in den Blättern von Rhus copallina nur die Inhaltsstoffe extrahiert und angereichert werden, die besonders vorteilhafte pharmakologische Eigenschaften haben. Eine Übersicht über die Vielzahl der chemischen Verbindungen in Rhus copallina sowie den Gehalt an diesen Stoffen in der Pflanze kann der oben genannten Dissertation von Annette Abhau entnommen werden. Durch das erfindungsgemäße Extraktionsverfahren werden aus diesen vielen Inhaltsstoffen, die in den Blättern vorhanden sind, nur bestimmte Inhaltsstoffe in bestimmten Mengen extrahiert. Diese Extraktion führt daher zu einer Zusammensetzung des Extrakts, welches die vorteilhaften pharmakologischen Eigenschaften aufweist, insbesondere die antiphlogistischen, anti-inflammatorischen, juckreizstillenden. adstringierenden, antiseptischen und antibakteriellen Eigenschaften sowie die vorteilhaften gastrointestinalen Wirkungen. Zu letzterem zählt die darmwandabdichtende Wirkung, die Erniedrigung einer erhöhten gastrointestinalen Permeabilität, eine Verlangsamung der Darmpassage, eine Erhöhung der Stuhlkonsistenz, die Verminderung einer gastrointestinalen Hypersekretion und toxinbindende Eigenschaften gegenüber enteropathogenen Keimen.

Die Blätter von Rhus copallina werden im Frühling oder im Sommer oder im Herbst geerntet. Gegebenenfalls kann auch eine Mischung von Blättern, die zu verschiedenen Jahreszeiten geerntet werden, verwendet werden. Beispielsweise können Blätter verwendet werden, die im Mai, Juni und/oder Oktober geerntet werden. Die Blätter können frisch verwendet werden, oder vor der Extraktion durch Trocknung haltbar gemacht werden. Es werden vorzugsweise nur die stiellosen Blätter ohne Stengel bzw. Zweige verwendet.

Die getrockneten oder frischen Blätter werden zerkleinert (geschnitten) und gegebenenfalls pulverisiert und gesiebt. Die zerkleinerten Blätter werden mit dem Extraktionsmittel versetzt. Die Extraktion wird über einen bestimmten Zeitraum und unter bestimmten Bedingungen durchgeführt. Durch die Extraktion werden die gewünschten Inhaltsstoffe aus den Blättern von Rhus copallina herausgelöst und angereichert, während unerwünschte Begleitstoffe in den Blättern verbleiben. Anschließend wird der Rückstand aus Blättern abgetrennt und es verbleibt der Rhus copallina Extrakt.

Offenbart wird ebenfalls ein Extrakt aus Rhus copallina, der nach diesem Verfahren hergestellt wird.

Die Extraktion kann beispielsweise mittels Mazeration oder Perkolation erfolgen. Bei der Mazeration werden die Blätter mit dem Extraktionsmittel eine definierte Zeit stehen gelassen, bis ein Gleichgewichtszustand erreicht ist (keine erschöpfende Extraktion). Bei der Perkolation werden die Blätter kontinuierlich mit dem Extraktionsmittel versetzt und der entstandene Extrakt abgetrennt.

Eine Offenbarung betrifft Rhus copallina Fluidextrakt, wobei die Extraktion für maximal 7 Tage, vorzugsweise 1 bis 5 oder 6 Tage, besonders bevorzugt 2, 3 oder 4 Tage durchgeführt wird oder gegebenenfalls andere bekannte Mazerationsverfahren. Besonders bevorzugt ist eine kurze und schnelle Extraktion, da auf diese Weise die in den Blättern enthaltenen Gerbstoffe nicht zu Gallussäure abgebaut werden. Eine schnelle Extraktion kann beispielsweise innerhalb von 10 Minuten oder bis zu 5 Stunden, vorzugsweise innerhalb einer Zeit von 30 Minuten und 3 Stunden, besonders bevorzugt etwa 1 Stunde durchgeführt werden. Besonders geeignete Reaktionsbedingungen für kurze und schnelle Extraktionsverfahren sind dem Fachmann bekannt.

Eine Offenbarung betrifft die Extraktion der Blätter bei erhöhter Temperatur, beispielsweise bei 30°C, 35°C, 40 °C oder 45 °C. Dadurch kann die Extraktionszeit verkürzt werden. Beispielsweise wird die Extraktion bei erhöhter Temperatur und unter Rückfluss für 2 oder 3 Stunden durchgeführt.

Nach der Extraktion wird der Rückstand (die Blätter) abgetrennt und der Extrakt gegebenenfalls eingeengt bzw. aufkonzentriert. Die Aufkonzentrierung kann beispielsweise durch schonendes Rotationsverdampfen bei 30 °C bis 40 °C erfolgen. Durch das Rotationsverdampfen kann der Extrakt bis fast zur Trockne eingeengt werden. Das Volumen des Extrakts kann beispielsweise um ein Drittel oder die Hälfte oder noch weiter reduziert werden.

Um den mengenmäßigen Bezug zwischen eingesetzten Blättern (Droge) und Extrakt deutlich zu machen, wird für einen Extrakt das Droge-Extrakt-Verhältnis angegeben. Das Droge-Extrakt-Verhältnis (DEV) gibt an, welche Ausgangsmenge an Droge (Drogengewicht, Trockenmasse) für die Bereitung einer bestimmten Menge des Extrakts eingesetzt wurde. Mit dem DEV kann bei der Dosierung immer auf die eingesetzte Drogenmenge umgerechnet werden und verschiedene Extrakte der Droge können in ihrer Qualität verglichen werden und Rückschlüsse auf die Anreicherung der Inhaltsstoffe gezogen werden.

Aus dem Extrakt von Rhuis copallina können beispielsweise Trockenextrakte z.B. für Tabletten hergestellt werden oder Fluidextrakte. Fluidextrakte (Flüssigextrakte) von Rhus copallina haben beispielsweise ein Verhältnis von Extraktivstoffen aus einem Teil Droge zu der Menge an Flüssigextrakt von 1:1. Fluidextrakte sind flüssige Drogenzubereitungen, bei denen für die Extraktion der Droge möglichst wenig Extraktionsflüssigkeit eingesetzt wird.

Bei dem verwendeten Extrakt aus den Blättern von Rhus copallina wird beispielsweise ein Droge / Extraktionsmittel-Verhältnis von 20 mg Droge zu 1 ml Lösungsmittel bzw. Extraktionsmittel, oder 15 g Droge in 150 ml Extraktionsmittel verwendet. In den Blättern waren je nach Jahreszeit allein bis zu 28 % Gerbstoffe (m/m) enthalten. Dadurch kann das DEV entsprechend variiert werden. Das DEV kann beispielsweise bei 3 : 1 bis 5 : 1 liegen, andere DEV sind erfindungsgemäß ebenfalls umfasst.

Eine weitere Offenbarung ist eine pharmazeutische Zusammensetzung, vorzugsweise in Form einer Salbe, einem Saft, einer Lotion, einer Lösung, einem Suppositorium, welche einen wässrig/alkoholischen Extrakt aus den Blättern von Rhus copallina umfasst. Die Salbe, Lotion, Lösung, Saft oder das Suppositorium können neben dem wässrig/alkoholischen Extrakt die üblichen Träger, Hilfs- und Zusatzstoffe umfassen.

Die pharmazeutischen Zusammensetzungen oder Zubereitungen können in Form von Dosierungseinheiten hergestellt werden. Dies bedeutet, dass die Zusammensetzungen in Form einzelner Teile, vorzugsweise Kapseln, Dragees und Ampullen vorliegen, deren Wirkstoffgehalt an dem erfindungsgemäßen Extrakt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge des erfindungsgemäßen Extrakts (Wirkstoff), die bei einer Applikation verabreicht wird und die gewöhnlich einer ganzen, einer halben, einem Drittel oder einem Viertel einer Tagesdosis entspricht.

Es kann die galenische Formulierung einer Calziummanteltablette gewählt werden, wie in EP1392337 offenbart.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen, wie a) Füll- und Streckmittel, z.B. Stärken, Milchzucker, Rohrzucker, Glukose, Mannit und Kieselsäure, b) Bindemittel, z.B. Carboxymethylcellulose, Alginate, Gelatine, Polyvinylpyrrolidon, c) Feuchthaltemittel, z.B. Glycerin, d) Sprengmittel, z.B. Agar-Agar, Calciumcarbonat und Natriumcarbonat, e) Lösungsverzögerer, z.B. Paraffin und f) Resorptionsbeschleuniger. z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Das Nahrungsergänzungsmittel kann ebenfalls in Form einer Zusammensetzung verabreicht werden, wie oben beschrieben. Das Nahrungsmittel kann einem beliebigen Nahrungsmittel oder Lebensmittel, einschließlich Wasser, zugesetzt werden.

Nahrungs- und Lebensmittel im Sinne dieser Erfindung sind insbesondere solche, wie nicht abschließend in EU-Richtlinie 2002/46/EG vom 10. Juni 2002 definiert.

## Patentansprüche

1. Arzneimittel oder Nahrungsergänzungsmittel umfassend einen wässrig-ethanolischen Extrakt aus Blättern von *Rhus copallina L.* zur Anwendung in einem Verfahren zur Behandlung von atopischer Dermatitis (Neurodermitis) oder Spasmen.

2. Arzneimittel oder Nahrungsergänzungsmittel umfassend einen wässrig-ethanolischen Extrakt aus Blättern von *Rhus copallina L.* zur Anwendung in einem Verfahren zur Behandlung von gastrointestinalen Erkrankungen, wobei eine gastrointestinale Erkrankung ausgewählt ist aus der Gruppe
a.) chronischen Magen- und/oder Darmfunktionsstörungen, wie die Regulation des Magen-Darm Traktes, allgemeine Verdauungsprobleme, Blähungen, Koliken und/oder gegen Verstopfung, Magenprobleme, wie Völlegefühl und Unwohlsein, Magen- und/oder Bauchschmerzen,
b.) chronisch-entzündliche Darmerkrankungen, wie Morbus Crohn oder Colitis ulcerosa, Gastroenteritis, Hamorrhoidalleiden, Reizdarm.

3. Arzneimittel oder Nahrungsergänzungsmittel zur Anwendung nach einem der vorhergehenden Ansprüche mittels einer oralen, dermalen und/oder mucosalen Darreichung.

4. Arzneimittel oder Nahrungsergänzungsmittel zur Anwendung nach einem der vorhergehenden Ansprüche, in Form einer Salbe, Saft, Lotion oder Zäpfchen, umfassend gängige Hilfs- und Zusatzstoffe.

## Claims

1. A pharmaceutical or dietary supplement comprising an aqueous-ethanolic extract of leaves of *Rhus copallina L.* for use in a method for treating atopic dermatitis (neurodermitis) or spasms.

2. A pharmaceutical or dietary supplement comprising an aqueous-ethanolic extract of leaves of *Rhus copallina L.* for use in a method for treating gastrointestinal disorders, wherein the gastrointestinal disorder is selected from the group
a.) chronic stomach and/or intestinal disturbances, such as the regulation of the gastrointestinal tract, general digestive problems, bloating, colic and/or constipation, stomach problems, such as a sensation of fullness and discomfort, stomach and/or abdominal pain,
b.) chronic-inflammatory intestinal disorders such as Crohn's disease or colitis ulcerosa, gastroenteritis, hemorrhoidal complaints, irritable colon.

3. A pharmaceutical or dietary supplement for use according to one of the preceding claims by means of oral, dermal and/or mucosal administration.

4. A pharmaceutical or dietary supplement for use according to one of the preceding claims, in the form of an ointment, juice, lotion, or suppository, comprising conventional auxiliary agents and additives.

## Revendications

1. Médicament ou complément alimentaire comprenant un extrait aqueux et éthanolique à base de feuilles de *Rhus copallina* L. pour une utilisation dans un procédé de traitement de dermatite atopique (neurodermite) ou de spasmes.

2. Médicament ou complément alimentaire comprenant un extrait aqueux et éthanolique à base de feuilles de *Rhus copallina* L. pour une utilisation dans un procédé de traitement de maladies gastro-intestinales, où une maladie gastrointestinale est choisie dans le groupe
a) des troubles fonctionnels stomacaux et/ou intestinaux chroniques, tels que la régulation du tractus stomacal-intestinal, des problèmes de digestion courants, des flatulences, des coliques et/ou de la constipation, de problèmes stomacaux, tels que des ballonnements et des malaises, des douleurs stomacales et/ou abdominales,
b) des maladies intestinales chroniques inflammatoires, telles que la maladie de Crohn ou la colite ulcéreuse, la gastroentérite, les hémorroïdes, l'intestin irritable.

3. Médicament ou complément alimentaire pour une utilisation selon l'une des revendications précédentes au moyen d'une administration orale, dermique et/ou mucosale.

4. Médicament ou complément alimentaire pour une utilisation selon l'une des revendications précédentes sous forme d'une pommade, d'un jus, d'une lotion ou de suppositoires, comprenant des substances auxiliaires et additives communes.
